(19) Europäisches Patentamt
European Patent Office
Office européen des brevets

(11) **EP 1 568 314 B1**

(12) **EUROPEAN PATENT SPECIFICATION**

(45) Date of publication and mention of the grant of the patent:
**06.07.2016 Bulletin 2016/27**

(51) Int Cl.:
*A61B 5/08* *(2006.01)*      *A61B 5/1455* *(2006.01)*
*A61B 5/022* *(2006.01)*      *A61B 5/024* *(2006.01)*
*A61B 5/0285* *(2006.01)*

(21) Application number: **05003571.6**

(22) Date of filing: **18.02.2005**

(54) **Blood pressure measurement apparatus and method**

Gerät und Verfahren zur Blutdruckmessung

Appareil et méthode de mesure de la pression sanguine

(84) Designated Contracting States:
**DE ES FR GB IT**

(30) Priority: **24.02.2004 JP 2004048404**

(43) Date of publication of application:
**31.08.2005 Bulletin 2005/35**

(73) Proprietors:
• **Omron Healthcare Co., Ltd.**
**Kyoto 617-0002 (JP)**
• **Kario, Kazuomi**
**Tochigi 329-0433 (JP)**

(72) Inventors:
• **Kario, Kazuomi**
**Kawachi-gun**
**Tochigi 329-0433 (JP)**
• **Shirasaki, Osamu**
**Muko-chi, Kyoto 617-0002 (JP)**

(74) Representative: **Kilian Kilian & Partner**
**Aidenbachstraße 54**
**81379 München (DE)**

(56) References cited:
EP-A- 0 123 313      EP-A- 0 445 809
EP-A- 0 875 200      EP-A- 1 127 538
US-A- 5 309 920

**Description**

BACKGROUND OF THE INVENTION

Field of the Invention

The present invention relates generally to blood pressure measurement apparatuses and methods and particularly to such apparatuses and methods driven by variation in physiological information other than blood pressure to determine when blood pressure measurement should be performed.

Description of the Background Art

In recent years a study using a mobile 24-hour blood pressure monitor (hereinafter referred to as "ambulatory blood pressure monitoring" (ABPM)) is revealing that in addition to daytime blood pressure, nighttime blood pressure is important in cardiovascular risk prediction and high blood pressure treatment. More specifically, if individual blood pressure information indicated by a blood pressure value attained when individual physiological condition varies during sleep, is obtained, the information would help to accurately assess individual cardiovascular risk and can thus be exploited in blood pressure control treatment.

The ABPM is characterized in that it is capable of measuring a subject's blood pressure intermittently for example for every 15 or 30 minutes and thus monitoring variation in blood pressure, whether or not the subject is sleeping or not, to serve as one of means for obtaining the above described blood pressure information.

In reality, however, it is difficult to apply the ABPM to general patients. First of all, the ABPM is expensive. Accordingly, it is affordable by only a limited number of medical facilities and accordingly, only a limited number of the ABPM can be used, so that in reality, it is used for only a limited number of patients. Furthermore, the measurement is repeated at intervals of a prescribed period of time. Accordingly in the measurement patients have their measurement sites, such as his/her upper arm, occluded and the apparatus also causes noise as it operates, which prevent the patients from sleeping, and they often feel uncomfortable or reject to use it. In addition, there is also a report that the uncomfortable feeling or mental stress varies a blood pressure value so that the actual blood pressure value can inaccurately be measured, as described for example in Calvo C, et al. Further evaluation of the ambulatory monitoring pressor effect ("ABPM effect") in patients with essential hypertension, J. Am. Hypertension, Vol. 16 No. 5, 2003, Lopez JE, Implications of the "ABPM effect" in hypertensive patients: Changes in the dipping pattern of blood pressure variability, J. Am. Hypertension, Vol. 16 No. 5, 2003, Hermida RC, Sampling requirements for ambulatory blood pressure monitoring in the diagnosis of hypertension, J. Am. Hypertension, Vol. 16 No. 5, 2003, Stenehjem AE, Gender and age differences in variability of ambulatory daytime pulse pressure, J. Am. Hypertension, Vol. 16 No. 5, 2003.

Furthermore, the ABPM is borrowed from medical facilities and used, rather than owned by an individual patient. As such, there is also a report that it is not used frequently and can thus provide insufficiently reliable measurement, as described for example by Murakami et. al., "Novelty Effect, Holiday Effect and Weekly Variation of 7-day Ambulatory (24-Hour) BP of Hypertensives" Circulation Journal, Japanese Circulation Society, Vol. 67, 2003.

In contrast, home-used blood pressure monitors recently, increasingly used do not have these disadvantages of the ABPM. However, they do not have a function capturing blood pressure variation.

Furthermore, there is also provided one that starts to measure blood pressure or similar biological information when it detects a stable condition during sleep, as described for example in Japanese Patent Laying-Open No. 8-131408.

Furthermore, there is also proposed a technique driven by a relative variation in pulse rate to start a blood pressure monitor, as described for example in Japanese Patent Laying-Open Nos. 62-155829 and 2-23940.

Blood pressure measurement employing the techniques of the ABPM as described in the above documents other than the Japanese patent applications still have difficulty in reproducing a resultant measurement. Japanese Patent Lay-ing-Open No. 8-131408 describes a method in which the fact is noted that when a person is sleeping his/her body movement is reduced and in response to a detection signal of a vibration sensor detecting body movement, when to start blood pressure measurement is timed. In other words, the measurement is not started as timed as based on variation in physiological information, and cannot provide a medically required blood pressure value reflecting variation in physiological information.

Japanese Patent Laying-Open Nos. 62-155829 and 2-23940 still have the following issue to be addressed: there is no concept that specifies a subject-unique physiological information variation range and physiological information's temporal variation is simply referred to for example to start a blood pressure monitoring. As such, they cannot capture blood pressure value in maximum, center and minimum ranges of physiological information and hence medically important.

Moreover, an automated sphygmomanometer is known from EP-A-1 127 538. The automated sphygmomanometer triggers a blood pressure determination upon detection of a significant change in the patient's heart rate variability (HRV). The HRV can be measured directly from the NIBP signal or, when a multiparameter monitor is used, the HRV can be measured from the ECG signal or the NIBP signal. HRV is continuously monitored and the baseline HRV is correlated with baseline blood pressure values. Changes in HRV are displayed continuously on a display so that the clinician can determine whether to initiate an NIBP measurement or, on the other hand, the NIBP measurement can be triggered automatically in response to a change in HRV without any intervention by the clinician. Alternatively, the patient monitor can "learn" the correlation between HRV and blood pressure changes and only alert the clinician when a significant change in HRV has taken place. The technique of the invention allows the clinician to monitor NIBP at less frequent intervals without the concern of missing physiologically significant changes in blood pressure and without the requirement of "check inflates" in a guard mode.

SUMMARY OF THE INVENTION

The present invention contemplates a blood pressure measurement apparatus and method which allows blood pressure measurement to be performed only when physiological information presents a subject unique variation to ensure necessary information in amount, quality and the like to collect blood pressure information. This object is achieved by a blood pressure measurement apparatus according to claims 1-3 and a corresponding method according to claim 5. Further advantageous embodiments of the invention are the subject-matter of the dependent claims.

The foregoing and other objects, features, aspects and advantages of the present invention will become more apparent from the following detailed description of the present invention when taken in conjunction with the accompanying drawings.

BRIEF DESCRIPTION OF THE DRAWINGS

Figs. 1A and 1B show a block configuration and an appearance, respectively, of a blood pressure measurement apparatus of a first example
Fig. 2 shows an example of a structure of a finger cuff
Fig. 3 represents an example of a waveform of a pulse wave signal.
Fig. 4 is a flow chart of a pulse rate storage stage in accordance with the first example
Fig. 5 is a flow chart of a blood pressure measurement stage in accordance with the first embodiment.
Fig. 6 shows an example of displaying a resultant measurement in accordance with the first example
Figs. 7, 8 and 9 are block diagrams showing configurations of the present blood pressure measurement apparatus of the invention and third and fourth examples.
Fig. 10 is a block diagram showing another configuration of the present blood pressure measurement apparatus of the fourth example
Figs. 11 and 12 are block diagrams showing configurations of the present blood pressure measurement apparatus of fifth and sixth examples respectively.

DESCRIPTION OF THE PREFERRED EXAMPLES

First example

The present example is based on variation in pulse rate, a type of continuous physiological information other than blood pressure, to time when blood pressure measurement should be started, as medically required. While pulse rate is herein used, it may be replaced with heart rate, which corresponds to a similar physiological phenomenon. If heart rate is used, an electrocardiograph is separately required.

Adopting a pulse rate or a heart rate as an index to time when blood pressure measurement should be started, has the following significance: a pulse rate or a heart rate is an index of the sympathetic nervous system's activity level and its increase is associated with a cardiovascular event risk. When a pulse rate or a heart rate varies, the sympathetic nervous system also varies. Accordingly, the blood pressure level at the time is specified and measured to attempt to obtain a novel blood pressure index that leads to cardiovascular risk prediction.

Note that while the present example adopts as the continuous physiological information a pulse rate based on variation in volume of a digital artery, other types of physiological information, i.e., blood oxygen saturation level, vascular elasticity level, pulse wave velocity, and the like, as will be described hereinafter, may be used.

Description of Configuration

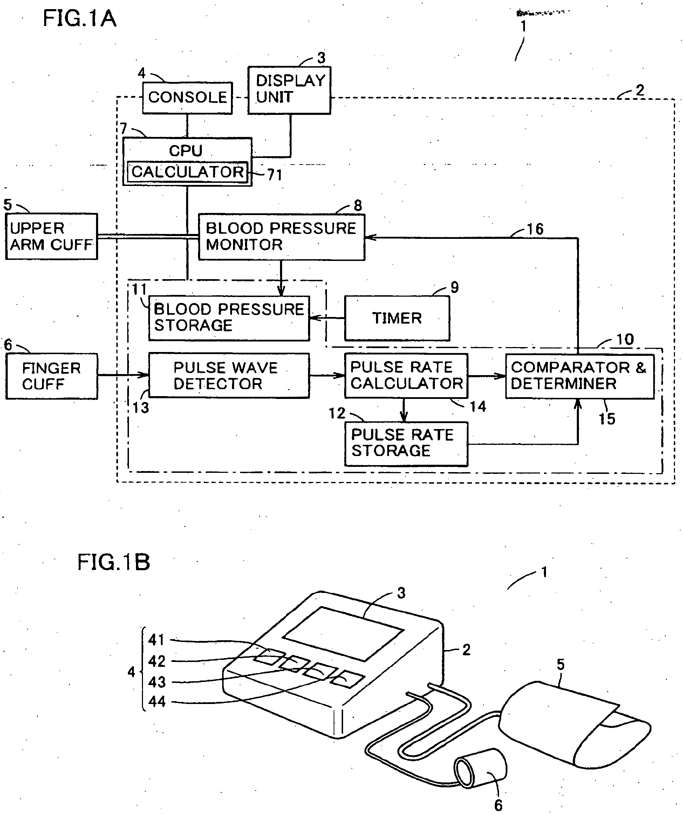

With reference to Figs. 1A and 1B, a blood pressure measurement apparatus 1 includes: a console 4 operated to provide a variety of instructions to a blood pressure measurement system, a system detecting continuous physiological information, and blood pressure measurement apparatus 1; a display unit 3 displaying a variety of information including resultant measurement, and a central processing unit (CPU) 7 intensively controlling and monitoring them.

The blood pressure measurement system includes a cuff 5 arranged external to a casing 2 indicated by a dotted line and wound around a subject's upper arm in blood pressure measurement, a blood pressure monitor 8 connected to cuff 5 via an air tube and operative in response to a pressure signal detected from cuff 5 to measure blood pressure and output a blood pressure value, a timer 9 counting time to output temporal information including a date and a time, and a blood pressure storage 11 receiving a blood pressure value from blood pressure monitor 8 to correlate each value with the temporal information output from timer 9 and store them. Blood pressure monitor 8 and cuff 5 will not be described as they are configured as well known. In accordance with an operation of console 4 by a user the contents of blood pressure storage 11 are read and displayed by display unit 3.

Console 4 includes a power supply switch 41 operated to turn on/off a power supply for blood pressure measurement apparatus 1, a measurement start switch 42 operated to issue instruction to start or stop measurement, a memory call switch 43 operated to issue instruction to read from the storage to display information of the storage at display unit 3, and a mode switch 44 operated to switch a mode of operation.

The continuous physiological information detection system includes a finger cuff 6 arranged external to casing 2 and attached to a subject's finger to continuously detect a pulse wave, a pulse wave detector 13 receiving a pulse wave signal from finger cuff 6 to recognize the pulse wave for each single beat, a pulse rate calculator 14 receiving an output from pulse wave detector 13 to calculate a pulse rate based on the received output, a pulse rate storage 12 storing the pulse rate calculated by pulse rate calculator 14, and a comparator and determiner 15 comparing the pulse rate stored in pulse rate storage 12 with the currently measured pulse rate and making a decision therefrom, and driven by the decision to output a start signal 16 instructing blood pressure monitor 8 to start blood pressure measurement.

In Fig. 1, blood pressure storage 11 and pulse rate storage 12, which are configured of memory, and pulse wave detector 13, pulse rate calculator 14 and comparator and determiner 15, which are provided in the form of a program, configure a processor 10. CPU 7 controls access to blood pressure storage 11 and pulse rate storage 12 and also controls execution of the program of pulse wave detector 13, pulse rate calculator 14 and comparator and determiner 15. CPU 7 has a calculator 71 provided in the form of a program executed by CPU 7.

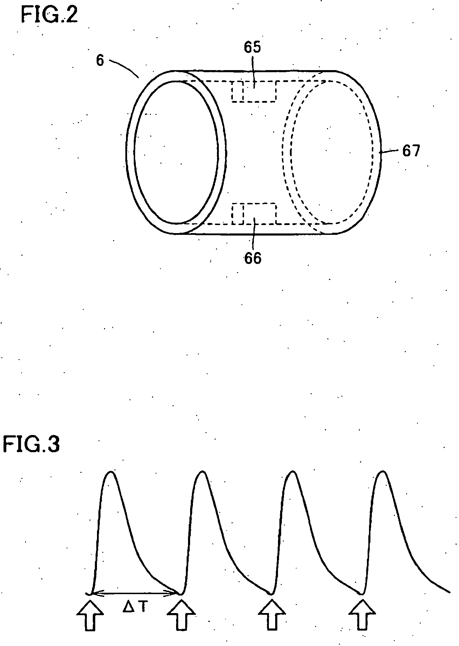

For a pulse wave sensor for blood pressure measurement apparatus 1 a variety of configurations and structures can be applied. Herein, finger cuff 6 shown in Fig. 2 is for example adopted. Finger cuff 6 is configured to include a cylindrical cuff 67, and a light emitting element 65 and a light receiving element 66 incorporated in cuff 67. When a subject has finger cuff 6 attached on his/her finger and light emitting element 65 illuminates the finger for example with an infrared beam of light, the finger transmits beam, which is detected by light receiving element 66. The finger's arterial volume repeats to increase and decrease in accordance with pulsation of blood pressure, and the amount (or intensity) of infrared radiation, which is more absorbable by blood hemoglobin, that is transmitted through the finger varies as the finger's arterial volume varies. Light receiving element 66 outputs to pulse wave detector 13 a variation in amount of the infrared radiation transmitted through the finger and received. The variation is represented for example by a signal representative for example of a variation in voltage. This signal is referred to as a pulse wave signal, such as shown for example in Fig. 3.

Pulse wave detector 13 receiving the pulse wave signal recognizes for each single beat the pulse wave's rising point (indicated in Fig. 3 by an arrow) represented by the pulse wave signal. Then, pulse rate calculator 14 measures a temporal interval $\Delta T$ between adjacent rising points of the pulse wave and calculates the number of beats per unit time, i.e., a pulse rate.

Pulse rate storage 12 can store pulse rate for example over a plurality of days. Furthermore, comparator and determiner 15 connects pulse rate storage 12, pulse rate calculator 14 and blood pressure monitor 8 together and compares a previous pulse rate stored in pulse rate storage 12 with a current pulse rate transmitted from pulse rate calculator 14 to determine whether the subject's current pulse rate is around a specified range relative to the previous pulse rate's variation range, more specifically, whether it is around a maximum value, an average value, or a minimum value. If a decision is made that the current pulse rate is around such a specified range, start signal 16 is output to blood pressure monitor 8. Blood pressure monitor 8 receives start signal 16 and responsively starts blood pressure measurement.

Description of Operation

In the present embodiment blood pressure measurement apparatus 1 is used in three stages, i.e., 1) the stage of measuring a pulse rate for a prescribed period of time to store data of a plurality of pulse rates to obtain a subject unique pulse rate variation range (the "pulse rate storage stage"), 2) the stage of measuring blood pressure, as based on the distribution of the data of the plurality of pulse rates stored, when a currently measured pulse rate approaches a specified range within the subject unique variation range (the "blood pressure measurement stage"), and 3) the stage of reading and outputting (or displaying) the stored pulse rate data and blood pressure data for study.

One important medical object of the present invention is to measure blood pressure at an instant during sleep when a physiological variation occurs. Accordingly, in the following description, for better understanding, the pulse rate storage and blood pressure measurement stages are both performed as blood pressure measurement apparatus 1 is used during sleep. While it is noted herein that it is known that blood pressure varies with autonomic nerve activity and that autonomic nerve activity during sleep and blood pressure variation attributed thereto have a medical significance, and blood pressure measurement apparatus I is accordingly used during sleep, although it may not be used during sleep. Note herein that autonomic nerve activity is obtained from physiological information for example of a type associated with cardiovascular risk.

Operation of Pulse Rate Data Storage Stage

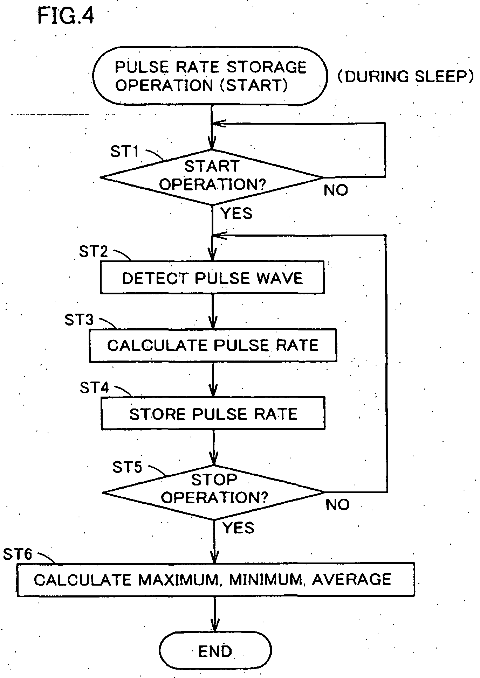

With reference to Fig. 4, the pulse rate storage step's operation will be described. A program in accordance with the Fig. 4 flow chart is stored in a memory (not shown) associated with CPU 7, and read and executed by CPU 7.

This stage only stores pulse rate data obtained by measuring a pulse wave. Accordingly, the user simply has finger cuff 6 attached thereon and operates mode switch 44 of console 4 to switch blood pressure measurement apparatus 1 to enter a pulse rate data storage mode and initiate blood pressure measurement apparatus 1. Note that blood pressure measurement apparatus 1 may be adapted to switch to enter a storage mode automatically performing a pulse rate data storage operation when there is no pulse rate data stored. In this scenario, finger cuff 6 is attached on a subject's finger for the sake of illustration.

For example if the latter storage mode is entered and measurement start switch 42 is operated to start blood pressure measurement apparatus 1, at step (ST) 1 CPU 7 recognizes that blood pressure measurement apparatus 1 is started in the storage mode. In subsequent ST2 CPU 7 instructs pulse wave detector 13 to detect a pulse wave's rising point for each beat from a pulse wave signal obtained from a pulse wave sensor of finger cuff 6. Then at ST3 CPU 7 instructs pulse rate calculator 14 to calculate a pulse rate for each beat (an amount converted to the number of beats of the pulse wave per unit time), and CPU 7 goes to ST4 to store data representing the pulse rate to pulse rate storage 12. Then at subsequent ST5 CPU 7 determines whether the user operates console 4 to stop blood pressure measurement apparatus 1 as he/she has got up. If not, CPU 7 returns to ST2 and thereafter ST2-ST4 are repeated for a pulse wave signal detected. Continuously detected pulse rate data can thus be stored to pulse rate storage 12.

If blood pressure measurement apparatus 1 is stopped, CPU 7 goes to ST6 to cause calculator 71 to calculate maximum, minimum and average pulse rates from the series of pulse rate data stored in pulse rate storage 12, stores data thereof to pulse rate storage 12, and thereafter ends the pulse rate storage operation.

In the above description, a pulse rate is stored for each beat to pulse rate storage 12. In general, however, a pulse wave has a cycle varying for each beat as the cycle is affected for example by arrhythmia, respiratory variation, and the like. As such, there is also a concern that such may result in a current, qualitative, physiological condition represented inaccurately to some extent. This can be resolved simply by providing pulse rate calculator 14 with an averaging function. More specifically, it can be done simply by adapting pulse rate calculator 14 to perform a moving averaging process on pulse rate data calculated for each beat for a few beats. For 5-point moving average, for example, the pulse rate for a beat of interest and those for the immediately preceding two beats and the immediately following two beats for a total of five beats are averaged out and stored as an average value in pulse rate of the beat of interest to pulse rate storage 12. This is a well-known averaging process and accordingly will not be described in detail.

Furthermore, while in the Fig. 4 procedure a pulse rate is continuously detected and its data is stored to pulse rate storage 12, the pulse rate may be detected intermittently at intervals of a prescribed period of time and stored to pulse rate storage 12.

Operation of Blood Pressure Measurement Stage

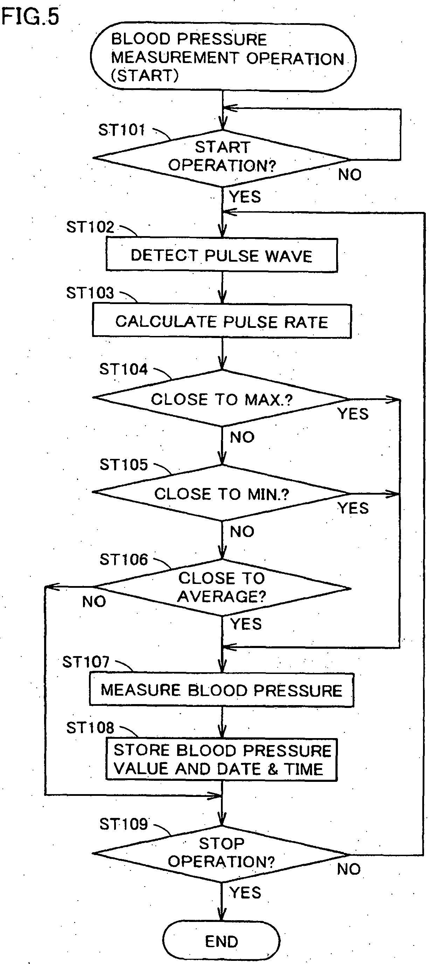

With reference to Fig. 5, the blood pressure measurement stage's operation will be described. A program in accordance with the Fig. 5 flow chart is stored to a memory (not shown) associated with CPU 7, and read and executed by CPU 7.

In this stage, blood pressure is measured around a specified relative level in a pulse rate variation range during sleep, i.e., around maximum, average and minimum pulse rate values in the range, as based on pulse rate data obtained at the pulse rate storage stage and stored to pulse rate storage 12, and resultant, measured blood pressure data is stored to blood pressure storage 11. If blood pressure data are thus accumulated in blood pressure storage 11 over one night (or while the subject sleeps once), the present stage's operation will also be performed at the subsequent night (or the next time when the subject sleeps).

Herein the Fig. 4 procedure is followed and pulse rate storage 12 has pulse rate data, and maximum, minimum and average values previously stored therein for the sake of illustration.

Initially, a subject has finger cuff 6 and upper arm cuff 5 attached thereto for pulse rate measurement and blood pressure measurement, respectively, and operates the console 4 mode switch 44 to set a mode of operation of the blood pressure measurement stage and operates measurement start switch 42 to start blood pressure measurement apparatus 1.

Initially CPU 7 goes to ST101 to recognize that blood pressure measurement apparatus 1 is started in the mode of operation of the blood pressure measurement stage. CPU 7 then goes to STs 102 and 103 to perform a pulse wave detection process and a pulse rate calculation process, respectively. These processes will not specifically be described as they are identical to an operation performed at the pulse rate storage stage including the averaging process previously described.

CPU 7 then goes to the following steps STs 104-106 to instruct comparator and determiner 15 to operate. In response, comparator and determiner 15 compares pulse rate data calculated from a currently measured pulse wave with a distribution of pulse rate data previously stored in pulse rate storage 12 to determine whether the pulse rate indicated by the pulse rate data indicates a value close to the maximum value, the minimum value or the average value:

Whether the pulse rate indicates a value close to the maximum value may be determined as follows: if pulse rate storage 12 has N previous pulse rate data stored therein and represented by PR (1), PR (2), ..., PR (N), their average value PRav and standard deviation PRsd are calculated by the following expressions:

$$PRav = \sum_{i=1}^{N} PR\ (i) / N \qquad\qquad \ldots (1)$$

$$PRsd = \left[ \sum_{i=1}^{N} \{PR\ (i) - PRav\}^2 / N \right]^{1/2} \qquad \ldots (2).$$

If a current pulse rate is represented by PR (0), whether PR (0) indicates a value close to the maximum value is determined by whether the following expression:

$$PR\ (0) > PRav + 2 \cdot PRsd \qquad\qquad \ldots (3)$$

is established (ST104).

Expression (3) means that when a current pulse rate exceeds a previous pulse rate average value plus the standard deviation multiplied by two a decision is made that the current pulse rate indicates a value close to the maximum value. A similar concept is applied to determine that the current pulse rate PR (0) indicates a value close to the minimum value by that the following equation:

$$PR\ (0) < PRav - 2 \cdot PRsd \qquad\qquad \ldots (4)$$

is established (ST105).

Furthermore, a decision made at ST106 as to whether the current pulse rate PR (0) indicates a value close to the average is made by whether pulse rate PR (0) falls within a range in value having upper and lower limits with a previous pulse rate average value serving as a center. This decision for example follows the following expression:

$$PRav - 0.2 \cdot PRsd < PR\ (0) < PRav + 0.2 \cdot PRsd \qquad \ldots (5).$$

If none of the STs 104-106 conditions are established, CPU 7 goes to a subsequent ST109. If any of the conditions is established, then CPU 7 goes to ST107. At ST107, comparator and determiner 15 outputs start signal 16 to blood pressure monitor 8, and blood pressure monitor 8 accordingly measures the subject's blood pressure. Subsequently at ST108 CPU 7 correlates with each other an obtained blood pressure measurement or systolic and diastolic blood pressure value data, a date, a time and other similar temporal information supplied from timer 9, a current pulse rate's data, and information of whether the current pulse rate is around the maximum value, the minimum value or the average value, and stores them to blood pressure storage 11.

At subsequent ST 109 CPU 7 determines whether console 4 is operated to stop the measurement. This is determined for example by whether measurement start switch 42 is operated. If not then CPU 7 returns to ST102 and the subsequent process is similarly repeated. If the operation to stop the measurement is performed, the series of steps ends.

For a period from ST101, performed to start blood pressure measurement apparatus 1, through to ST109, performed to stop it, whenever STs 102 and 103 are performed to measure to a pulse rate a decision is made as to when to measure blood pressure or blood pressure is measured. Alternatively, a pulse rate may be measured during a prescribed period of time after blood pressure measurement apparatus 1 is started and before it is stopped, and for each measured pulse rate a decision as to when to measure blood pressure may be made or blood pressure may be measured.

In the above description, an operation storing to pulse rate storage 12 a pulse rate continuously measured at the blood pressure measurement stage is not included and the pulse rate is used simply to time when blood pressure monitor 8 should be started for blood pressure measurement. If the blood pressure measurement operation, and storing pulse rate data to pulse rate storage 12, as performed in the pulse rate storage stage, are simultaneously performed, however, data stored indicating the subject's pulse rate variation range can be more reliable. This can be achieved simply by further introducing a step after the Fig. 5 ST103 to perform a process to store a pulse rate.

Data Read Operation

In this operation the data stored in the Fig. 4 pulse rate storage stage and the Fig. 5 blood pressure measurement stage to pulse rate storage 12 and blood pressure storage 11 are read by CPU 7 in response to memory call switch 43 being operated, and blood pressure data is displayed by display unit 3, as correlated with pulse rate variation.

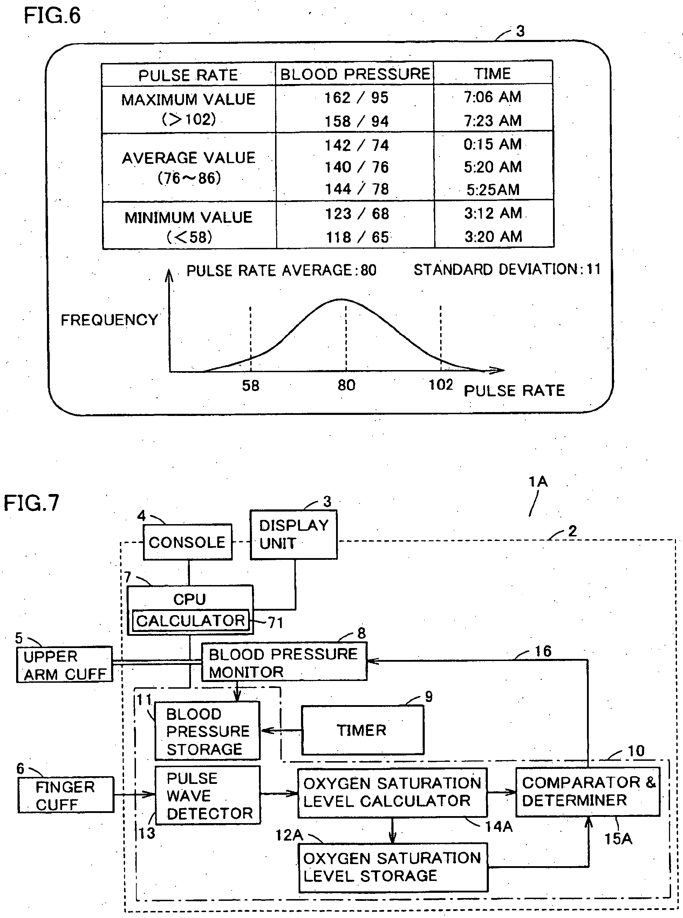

It is displayed for example as shown in Fig. 6. Initially, display unit 3 has a screen with an upper portion displaying a blood pressure value and its measurement time correlated with each other, as categorized in the form of a list by whether a pulse rate associated with the measured blood pressure falls within the maximum value's range, the average value's range, or the minimum value's range. While blood pressure is measured, more than one pulse rate may satisfy a condition corresponding to any of the ranges of the maximum, average and minimum values, respectively, and any blood pressure values measured whenever that condition is established and their measurement times are displayed.

Furthermore on the same screen at a lower portion is displayed a distribution of a pulse rate obtained at the pulse rate storage stage, as processed by CPU 7, statistically and diagrammatically. The horizontal axis represents pulse rate, and all data's frequency graph (a histogram) and the average, maximum and minimum values' threshold values, as indicated in the graph by vertical broken lines, and a pulse rate average and a pulse rate standard deviation by numerical representation are displayed.

In the present embodiment a pulse rate is continuously monitored as physiological information associated with a risk for cardiovascular system that allows a measurement which does not prevent a subject from sleeping, and when the physiological information presents a desired variation, i.e., when the pulse rate indicates a value close to the maximum, minimum or average value, the subject's blood pressure is measured and the resultant, measured blood pressure level and variation are presented via display unit 3 to help the subject to assess and control his/her blood pressure for himself/herself and doctors to select among antihypertensive treatments to allow earlier, optimal antihypertensive control and treatment. A resultant measurement and a treatment that is selected are for example as follows:

In Fig. 6 at the lower portion the histogram indicates a distribution of a pulse rate used as information to start blood pressure measurement, and threshold value information indicating a level for which a blood pressure measurement is started as the pulse rate increases/decreases to the level. It can be understood that if threshold values are spaced closely relative to the distribution, a large amount of data exist outer than that, and blood pressure measurement has frequently been initiated, and that in contrast, if the threshold values are spaced too wide, blood pressure measurement has not been initiated as often as expected, or blood pressure value data is insufficient.

In Fig. 6 at the upper portion the list shows blood pressure values as measured by the start operation indicated by the data at the lower portion. While the pulse rate's increase/decrease only provides no more than a conjecture that blood pressure increases/decreases, the upper portion's blood pressure value records and presents an extent to which it has in effect increased/decreased. Doctors can understand from this information for example whether an antihypertensive drug effectively/ineffectively works, when blood pressure increased/decreased, and other information to more specifically understand morbidity so that they can optimally prescribe drugs in amount, type and the like.

Invention

In the present invention variation in blood oxygen saturation level, a type of continuous physiological information other than blood pressure, is referred to to time when to start measurement of blood pressure, as medically required.

Adopting blood oxygen saturation level as an index to time when blood pressure measurement should be started, has the following significance: blood oxygen saturation level drops when sleep apnea syndrome or similar no- or shallow-breathing occurs. After breathing stops, a blood pressure level rapidly increases. Accordingly, the blood pressure at the time is measured to attempt to obtain a novel blood pressure index leading to cardiovascular risk prediction.

Configuration

Fig. 7 shows in a block a configuration of a blood pressure measurement apparatus 1A of the invention. Blood pressure measurement apparatus 1 of Fig. 1 A and blood pressure measurement apparatus 1 A differ in that blood pressure measurement apparatus 1 includes pulse rate storage 12, pulse rate calculator 14 and comparator and determiner 15, whereas blood pressure measurement apparatus 1A instead includes an oxygen saturation level storage 12A, an oxygen saturation level calculator 14A, and a comparator and determiner 15A. The other components are identical to those of blood pressure measurement apparatus 1.

Blood oxygen saturation level is obtained as follows: finger cuff 6 pinches a finger tip and light emitting element 65 emits two types of light different in wavelength to expose the finger thereto. The finger transmits light, which is received by light receiving element 66 and its variation in amount is detected by pulse wave detector 13 as a pulse wave signal and therefrom oxygen saturation level calculator 14A following a known procedure calculates a blood oxygen saturation level. The calculated blood oxygen saturation level's data is sequentially stored to oxygen saturation level storage 12A and when the measurement completes, relative, maximum, average and minimum blood oxygen saturation level values' data are calculated, similarly as has been described in the first example and stored to oxygen saturation level storage 12A. Comparator and determiner 15A compares a currently measured blood oxygen saturation level with the maximum, average and minimum values stored in oxygen saturation level storage 12A and in accordance therewith outputs start signal 16. Thereafter, blood pressure measurement is performed in a procedure similar to that described in the first example

Third example

In the present invention arterial elasticity variation, a type of continuous physiological information other than blood pressure, is referred to to time when to start measurement of blood pressure, as medically required.

Adopting arterial elasticity as an index to time when blood pressure measurement should be started, has the following significance: for people of advanced age, an increase in systolic blood pressure directly links to a risk of apoplexy and such increase in level depends on arterial elasticity. Increased vascular elasticity provides a rapidly increased blood pressure level, and the blood pressure level at the time is measured to attempt to obtain a novel blood pressure index leading to cardiovascular risk prediction.

Configuration

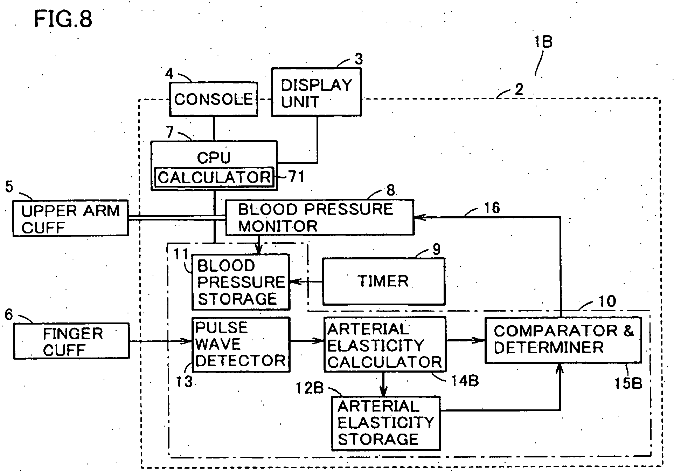

Fig. 8 shows in block a configuration of a blood pressure measurement apparatus 1B of the present embodiment. The Fig. 1A blood pressure measurement apparatus 1 and blood pressure measurement apparatus 1B differ in that the former includes pulse rate storage 12, pulse rate calculator 14 and comparator and determiner 15, whereas the latter instead includes an arterial elasticity storage 12B, an arterial elasticity calculator 14B, and a comparator and determiner 15B. The other components are identical to those of blood pressure measurement apparatus 1.

Arterial elasticity indicates a level in elasticity of a peripheral arteries and can be detected from an amount of a feature of a reflected wave obtained as a wave of blood pressure delivered from a heart is reflected by the peripheral arteries. The amount of the feature of the reflected wave can be calculated by arterial elasticity calculator 14B in a known procedure from a waveform of a pulse wave output from pulse wave detector 13. The calculated amount of the feature is sequentially stored as arterial elasticity to arterial elasticity storage 12B and when the measurement completes, relative, maximum, average and minimum values' data are calculated, similarly as has been described in the first embodiment, and stored to arterial elasticity storage 12B. Comparator and determiner 15B compares a currently measured arterial elasticity's data with the maximum, average and minimum values' data stored in arterial elasticity storage 12B and in accordance with a result thereof outputs start signal 16. Thereafter, blood pressure measurement is performed through a procedure similar to that described in the first example

Fourth example

In the present example of pulse wave velocity or arterial compliance, a type of continuous physiological information other than blood pressure, a variation in pulse wave velocity or arterial compliance is referred to to time when to start measurement of blood pressure, as medically required.

Adopting pulse wave velocity, arterial compliance or the like as an index to time when blood pressure measurement should be started, has the following significance; it is known that pulse wave velocity, arterial compliance and the like are associated with a cardiovascular event risk. When pulse wave velocity, arterial compliance or the like increases in level, the current blood pressure level is measured to attempt to obtain a novel blood pressure index leading to cardiovascular risk prediction.

Configuration

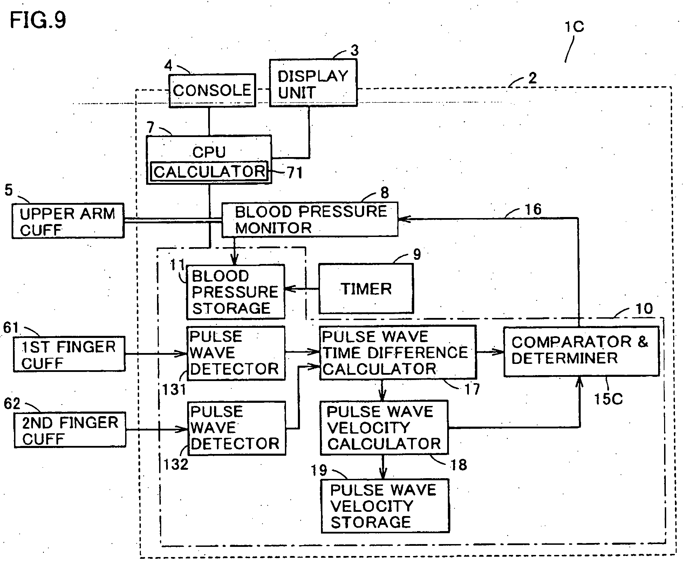

Fig. 9 shows in a block a configuration of a blood pressure measurement apparatus 1C of the present example that utilizes pulse wave velocity. The Fig. 1A blood pressure measurement apparatus 1 and blood pressure measurement apparatus 1C differ in that the former includes finger cuff 6, whereas the latter instead includes first and second cuffs 61 and 62 attached to a site for measurement to output a pulse wave signal, and that processor 10 includes blood pressure storage 11, pulse wave detectors 131 and 132 receiving pulse wave signals from the first and second cuffs 61 and 62, respectively, to detect a pulse wave for each beat, a pulse wave time difference calculator 17 receiving pulse waves from pulse wave detectors 131 and 132 to calculate the pulse waves' time difference, a pulse wave velocity calculator 18 using the calculated time difference and a distance between different measurement sites to calculate a pulse wave velocity data, a pulse wave velocity storage 19 sequentially storing the calculated pulse wave velocity data, and a comparator and determiner 15C. The other components are identical to those of blood pressure measurement apparatus 1. Measuring a pulse wave velocity requires attaching the first and second cuffs 61 and 62 at sites for measurement spaced by a distance. Accordingly, the first and second cuffs 61 and 62 are attached for example to a subject's upper arm and a finger associated therewith, or his/her upper arm and lower extremity. If the first and second cuffs 61 and 62 are attached to predetermined sites, their distance can uniquely be specified as the sites have therebetween a distance which does not significantly vary between individuals. Note that a distance between sites for measurement may be designated by inputting it via a key.

When the pulse wave velocity data is calculated and completely stored to pulse wave velocity storage 19, relative, maximum, average and minimum values' data are calculated, similarly as has been described in the first example, and stored to pulse wave velocity storage 19. Comparator and determiner 15C compares a currently measured pulse wave velocity data with those of the maximum, average and minimum values stored in pulse wave velocity storage 19 and in accordance with a result thereof outputs start signal 16. If thereafter, blood pressure measurement is performed through a procedure similar to that described in the first example.

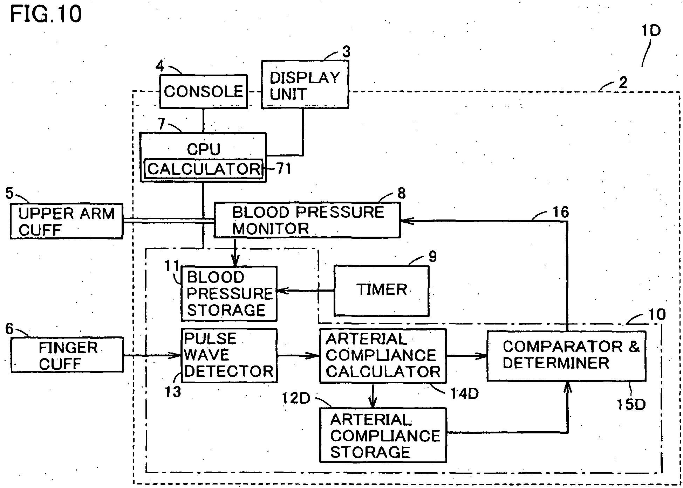

Fig. 10 shows in a block a configuration of a blood pressure measurement apparatus 1D of the present example. The Fig. 1A blood pressure measurement apparatus 1 and blood pressure measurement apparatus 1D differ in that the former includes pulse rate storage 12, pulse rate calculator 14 and comparator and determiner 15, whereas the latter instead includes an arterial compliance storage 12D, an arterial compliance calculator 14D, and a comparator and determiner 15D. The other components are identical to those of blood pressure measurement apparatus 1.

Arterial compliance can be calculated from a blood pressure waveform recorded from a finger continuously, i.e., a pulse waveform based on a pulse wave signal detected from finger cuff 6. This calculation's procedure is known and will not be described. Arterial compliance calculator 14D calculates arterial compliance's data, which is sequentially stored to arterial compliance storage 12D, and when the measurement completes, relative, maximum, average and minimum values' data are calculated, similarly as has been described in the first example and stored to arterial compliance storage 12D. Comparator and determiner 15D compares a currently measured arterial compliance's data with those of the maximum, average and minimum values stored in arterial compliance storage 12D, and in accordance with a result thereof outputs start signal 16. Thereafter, blood pressure measurement is performed through a procedure similar to that described in the first example.

Fifth example

In the present example a variation in respiratory cycle, a type of continuous physiological information other than blood pressure, is referred to to time when to start measurement of blood pressure, as medically required.

Adopting respiratory cycle has an index to time when to start blood pressure measurement, has the following significance: respiratory cycle in nighttime serves as an index of sleep apnea syndrome and in wakefulness it is significantly affected by mentally stressed condition. A blood pressure level provided when a respiratory cycle varies is measured to attempt to obtain a novel blood pressure index leading to cardiovascular risk protection. While a respiratory cycle is herein employed, it may be replaced with a respiration rate of frequency. Its significance is the same as that of respiratory cycle.

Configuration

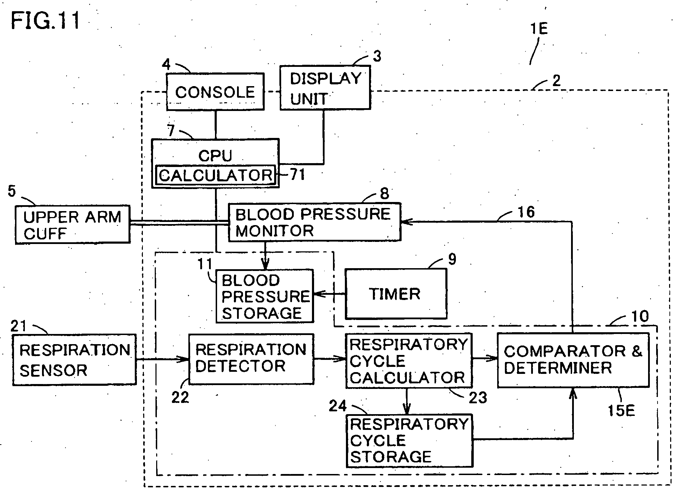

Fig. 11 shows in a block a configuration of a blood pressure measurement apparatus 1E of the present example that utilizes respiratory cycle. The Fig. 1A blood pressure measurement apparatus 1 and blood pressure measurement apparatus 1E differ in that the former has finger cuff 6 whereas the latter instead has a respiration sensor 21, and that processor 10 includes blood pressure storage 11, a respiration detector 22 detecting a subject's respiration, a respiratory cycle calculator 23 calculating data of the cycle of the respiration detected by respiration detector 22, a respiratory cycle storage 24 storing the calculated respiratory cycle data, and a comparator and determiner 15E. The other components are identical to those of blood pressure measurement apparatus 1.

When the respiratory cycle data is calculated and completely stored to respiratory cycle storage 24, relative, maximum, average and minimum values' data are calculated, similarly as has been described in the first example, and stored to respiratory cycle storage 24. Comparator and determiner 15E compares a currently measured respiratory cycle data with those of the maximum, average and minimum values stored in respiratory cycle storage 24, and in accordance with a result thereof outputs start signal 16. Thereafter, blood pressure measurement is performed through a procedure similar to that described in the first example.

Sixth example

In the present example a variation in blood pressure waveform, a type of continuous physiological information other than blood pressure, is referred to to time when to start measurement of blood pressure, as medically required.

Blood pressure waveform corresponds to a waveform of a signal obtained by measuring a physical quantity derived from an arterial variation attributed to arterial, internal pressure or an amount of blood flowing through an artery. Adopting blood pressure waveform as an index to time when to start blood pressure measurement, has the following significance: a blood pressure waveform has a large amount of biological information affected by peripheral vascular systolic level and cardiac function. By analyzing the waveform, an index (an augmentation index (AI) for example, is obtained, and when the index varies, blood pressure is measured to attempt to obtain a novel blood pressure index leading to cardiovascular risk prediction.

Configuration

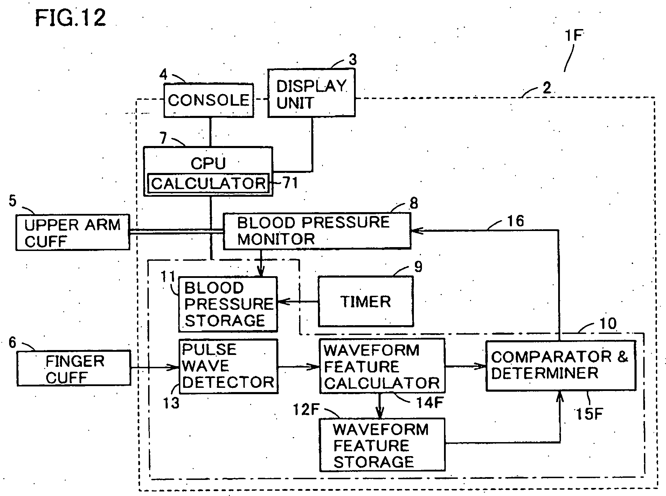

Fig. 12 shows in a block a configuration of a blood pressure measurement apparatus 1F of the present example. The Fig. 1 blood pressure measurement apparatus 1 and blood pressure measurement apparatus 1F differ in that the former includes pulse rate storage 12, pulse rate calculator 14 and comparator and determiner 15, whereas the latter instead includes a waveform feature storage 12F, a waveform feature calculator 14F and a comparator and determiner 15F. The other components are identical to those of blood pressure measurement apparatus 1.

The amount of a feature such as AI of a blood pressure waveform, i.e., a waveform of a pulse wave detected by pulse wave detector 13, can be calculated by waveform feature calculator 14F through a known procedure from the waveform of the pulse wave output from pulse wave detector 13. Data of the amount of the feature calculated is sequentially stored to waveform feature storage 12F and when the measurement completes, relative, maximum, average and minimum values' data are calculated, similarly as has been described in the first example and stored to waveform feature storage 12F. Comparator and determiner 15F compares data of an amount of a feature of a waveform currently measured with those of the maximum, average and minimum values stored in waveform feature storage 12F, and in accordance with a result thereof outputs start signal 16. Thereafter, blood pressure measurement is performed through a procedure similar to that described in the first example.

In the above examples blood pressure is measured only at a temporal point medically required as based on physiological information of a type other than blood pressure so that if the measurement is performed for a sleeping subject, the subject can minimally be prevented from sleeping, and blood pressure information required for diagnosis can be obtained without sacrificing it in quantity, quality and the like.

Although the present invention has been described and illustrated in detail, it is clearly understood that the same is by way of illustration and example only and is not to be taken by way of limitation, the spirit and scope of the present invention being limited only by the terms of the appended claims.

**Claims**

1. A blood pressure measurement apparatus (1; 1A; 1B; 1C; 1D; 1E; 1F) comprising:

blood pressure measurement means (8) adapted to measure a subject's blood pressure;

physiological information measurement means (6, 13, 14) adapted to measure physiological information which is blood oxygen saturation level;

physiological information storage means (12) adapted to store a series of values of said physiological information in level as measured by said physiological information measurement means (6, 13, 14) for a predetermined period of time, said physiological information storage means (12) being adapted to intermittently or continuously store said values of said physiological information in level as measured for said predetermined period of time; and determination means (7, 15) adapted to determine whether a value of said physiological information as currently measured by said physiological information measurement means (6, 13, 14) for determination of initiation of measurement of said subject's blood pressure exceeds a threshold value, wherein said blood pressure measurement means (8) is adapted to, when said determination means (15) determines that the value of said physiological information as currently measured exceeds the threshold value, be initiated to measure said subject's blood pressure, wherein:

said determination means (7, 15) includes calculation means (71) adapted to calculate an average value and a standard deviation of measured values of said series of physiological information previously stored; and

when said physiological information currently measured for determination of initiation of said blood pressure measurement provides a value exceeding said threshold value obtained from said average value and said standard deviation calculated by said calculation means (71), said determination means (7, 15) is adapted to determine that the provided value exceeds the threshold value and said blood pressure measurement means (8) is initiated.

2. A blood pressure measurement apparatus (1; 1A; 1B; 1C; 1D; 1E; 1F) comprising:

blood pressure measurement means (8) adapted to measure a subject's blood pressure;
physiological information measurement means (6, 13, 14) adapted to measure physiological information which is blood oxygen saturation level;
physiological information storage means (12) adapted to store a series of values of said physiological information in level as measured by said physiological information measurement means (6, 13, 14) for a predetermined period of time, said physiological information storage means (12) being adapted to intermittently or continuously store said values of said physiological information in level as measured for said predetermined period of time; and determination means (7, 15) adapted to determine whether a value of said physiological information as currently measured by said physiological information measurement means (6, 13, 14) for determination of initiation of measurement of said subject's blood pressure is smaller than a threshold value, wherein said blood pressure measurement means (8) is adapted to, when said determination means (15) determines that the value of said physiological information as currently measured is smaller than the threshold value, be initiated to measure said subject's blood pressure, wherein:

said determination means (7, 15) Includes calculation means (71) adapted to calculate an average value and a standard deviation of measured values of said series of physiological information previously stored; and

when said physiological information currently measured for determination of initiation of said blood pressure measurement provides a value smaller than said threshold value obtained from said average value and said standard deviation calculated by said calculation means (71), said determination means (7, 15) is adapted to determine that the provided value is smaller than the threshold value and said blood pressure measurement means (8) is initiated.

3. A blood pressure measurement apparatus (1; 1A; 1B; 1C; 1D; 1E; 1F) comprising:

blood pressure measurement means (8) adapted to measure a subject's blood pressure;
physiological information measurement means (6, 13, 14) adapted to measure physiological information which is blood oxygen saturation level;
physiological information storage means (12) adapted to store a series of values of said physiological information in level as measured by said physiological information measurement means (6, 13, 14) for a predetermined period of time, said physiological information storage means (12) being adapted to intermittently or continuously store said values of said physiological Information in level as measured for said predetermined period of time; and determination means (7, 15) adapted to determine whether a value of said physiological information as currently measured by said physiological information measurement means (6, 13, 14) for determination of initiation of measurement of said subject's blood pressure is existing between prescribed upper and lower limits, wherein said blood pressure measurement means (8) is adapted to, when said determination means (15) determines that the value of said physiological information as currently measured is existing between the prescribed upper and lower limits, be initiated to measure said subject's blood pressure, wherein:

said determination means (7, 15) includes calculation means (71) adapted to calculate an average value and a standard deviation of measured values of said series of physiological information previously stored; and

when said physiological information measured for said blood pressure measurement provides a value existing between the prescribed upper and lower limits obtained from said average value and said standard deviation calculated by said calculation means (71), said determination means (7, 15) is adapted to determine that the provided value is existing between the prescribed upper and lower limits and said blood pressure measurement means (8) is initiated.

4.  The blood pressure measurement apparatus of claim 1, 2 or 3, further comprising:

blood pressure storage means (11) correlating and storing a blood pressure value measured whenever said blood pressure measurement means (8) provides measurement, temporal information indicating when the blood pressure is measured, and information of said specified level determined by said determination means; and display means (3) displaying a content of said blood pressure storage means (11).

5.  A blood pressure measurement method comprising the steps of:

previously measuring physiological information which is blood oxygen saturation level (ST2, ST3); intermittently or continuously storing a series of said physiological information in level as previously measured for a predetermined period of time; currently measuring physiological information which is blood oxygen saturation level (ST102, ST103); and determining whether a value of said physiological information as currently measured at the step of currently measuring for determination of initiation of measurement of said subject's blood pressure exceeds a maximum threshold value, is smaller than a minimum threshold value or exists between prescribed upper and lower limits, wherein when a decision is made at the step of determining (ST104, ST105, ST106) that the value of said physiological information as currently measure either exceeds the maximum threshold value, either is smaller than the minimum threshold value or exists between said prescribed upper and lower limits, a measurement of said subject's blood pressure starts, wherein:

said step of determining includes calculating an average value and a standard deviation of previously measured values of said series of physiological information previously stored; and

when said physiological information currently measured for determination of initiation of said blood pressure measurement provides a value exceeding said maximum threshold value obtained from said average value and said standard deviation calculated in said calculating step, a decision is made at the step of determining that the provided value exceeds said maximum threshold value and a measurement of said subject's blood pressure starts, or

when said physiological information currently measured for determination of initiation of said blood pressure measurement provides a value smaller than said minimum threshold value obtained from said average value and said standard deviation calculated in said calculating step, a decision is made at the step of determining that the provided value is smaller than said minimum threshold value and a measurement of said subject's blood pressure starts, or

when said physiological information currently measured for determination of initiation of said blood pressure measurement provides a value existing between said prescribed upper and lower limits obtained from said average value and said standard deviation calculated in said calculating step, a decision is made at the step of determining that the provided value exists between said prescribed upper and lower limits and a measurement of said subject's blood pressure starts.

**Patentansprüche**

1.  Blutdruckmessvorrichtung (1; 1A; 1B; 1C; 1D; 1E; 1F), umfassend:

# EP 1 568 314 B1

eine Blutdruckmesseinrichtung (8), die eingerichtet ist, einen Blutdruck eines Probanden zu messen;
eine Messeinrichtung (6, 13, 14), die eingerichtet ist, eine physiologische Information, die ein Blutsauerstoffsättigungsniveau ist, zu messen;
eine Speichereinrichtung (12), die eingerichtet ist, eine Reihe von Werten der physiologischen Information hinsichtlich deren Höhe wie von der Messeinrichtung (6, 13, 14) gemessen für eine vorbestimmte Zeitperiode zu speichern, wobei die Speichereinrichtung (12) eingerichtet ist, die Werte der physiologischen Information hinsichtlich deren Höhe diskontinuierlich oder kontinuierlich wie gemessen für die vorbestimmte Zeitperiode zu speichern; und
eine Bestimmungseinrichtung (7, 15), die eingerichtet ist, zu bestimmen, ob ein Wert der physiologischen Information, der gegenwärtig von der Messeinrichtung (6, 13, 14) gemessen wird, zum Bestimmen einer Auslösung der Messung des Blutdruckes des Probanden einen Schwellenwert überschreitet, wobei die Blutdruckmesseinrichtung (8) eingerichtet ist, die Blutdruckmessung des Probanden auszulösen, wenn die Bestimmungseinrichtung (15) bestimmt, dass der gegenwärtig gemessene Wert der physiologischen Information den Schwellenwert überschreitet, wobei:

die Bestimmungseinrichtung (7, 15) eine Berechnungseinrichtung (71) aufweist, die eingerichtet ist, einen Durchschnittswert und eine Standardabweichung der gemessenen Werte der Reihe von Werten der physiologische Information, die vorher gespeichert ist, zu berechnen; und
wobei, wenn die gegenwärtig gemessene physiologische Information zur Bestimmung der Auslösung der Blutdruckmessung gegenwärtig einen Wert liefert, der den Schwellenwert, der von dem Durchschnittswert und der Standardabweichung, die von der Berechnungseinrichtung (71) berechnet werden, erhalten wird, überschreitet, die Bestimmungseinrichtung (7, 15) eingerichtet ist, zu bestimmen, dass der gelieferte Wert den Schwellenwert überschreitet und die Blutdruckmesseinrichtung (8) initiiert wird.

2. Blutdruckmessvorrichtung (1; 1A; 1B; 1C; 1D; 1E; 1F), umfassend:

eine Blutdruckmesseinrichtung (8), die eingerichtet ist, einen Blutdruck eines Probanden zu messen;
eine Messeinrichtung (6, 13, 14), die eingerichtet ist, eine physiologische Information, die ein Blutsauerstoffsättigungsniveau ist, zu messen;
eine Speichereinrichtung (12), die eingerichtet ist, eine Reihe von Werten der physiologischen Information hinsichtlich deren Höhe wie von der Messeinrichtung (6, 13, 14) gemessen für eine vorbestimmte Zeitperiode zu speichern, wobei die Speichereinrichtung (12) eingerichtet ist, die Werte der physiologischen Information hinsichtlich deren Höhe diskontinuierlich oder kontinuierlich wie gemessen für die vorbestimmte Zeitperiode zu speichern; und
eine Bestimmungseinrichtung (7, 15), die eingerichtet ist, zu bestimmen, ob ein Wert der physiologischen Information wie gegenwärtig von der Messeinrichtung (6, 13, 14) gemessen zum Bestimmen einer Auslösung der Messung des Blutdruckes des Probanden kleiner als ein Schwellenwert ist, wobei die Blutdruckmesseinrichtung (8) eingerichtet ist, die Blutdruckmessung des Probanden auszulösen, wenn die Bestimmungseinrichtung (15) bestimmt, dass der gegenwärtig gemessene Wert der physiologischen Information kleiner als der Schwellenwert ist, wobei:

die Bestimmungseinrichtung (7, 15) eine Berechnungseinrichtung (71) aufweist, die eingerichtet ist, einen Durchschnittswert und eine Standardabweichung der gemessenen Werte der Reihe von Werte der physiologische Informationen, die vorher gespeichert sind, zu berechnen; und
wobei, wenn die gemessene physiologische Information zur Bestimmung der Auslösung der Blutdruckmessung gegenwärtig einen Wert liefert, der kleiner als der Schwellenwert, der von dem Durchschnittswert und der Standardabweichung, die von der Berechnungseinrichtung (71) berechnet werden, erhalten wird, ist, die Bestimmungseinrichtung (7, 15) eingerichtet ist, zu bestimmen, dass der gelieferte Wert kleiner als der Schwellenwert ist und die Blutdruckmesseinrichtung (8) initiiert wird.

3. Blutdruckmessvorrichtung (1; 1A; 1B; 1C; 1D; 1E; 1F), umfassend:

eine Blutdruckmesseinrichtung (8), die eingerichtet ist, einen Blutdruck eines Probanden zu messen;
eine Messeinrichtung (6, 13, 14), die eingerichtet ist, eine physiologische Information, die ein Blutsauerstoffsättigungsniveau ist, zu messen;
eine Speichereinrichtung (12), die eingerichtet ist, eine Reihe von Werten der physiologischen Information hinsichtlich deren Höhe wie von der Messeinrichtung (6, 13, 14) gemessen für eine vorbestimmte Zeitperiode, wobei die Speichereinrichtung (12) eingerichtet ist, die Werte der physiologischen Information hinsichtlich deren Höhe wie gemessen diskontinuierlich oder kontinuierlich für die vorbestimmte Zeitperiode zu speichern; und eine Bestimmungseinrichtung (7, 15), die eingerichtet ist, zu bestimmen, ob sich ein Wert der physiologischen Information, der gegenwärtig von der Messeinrichtung (6, 13, 14) gemessen wird, zum Bestimmen einer Auslösung der Messung des Blutdruckes des Probanden zwischen vorgeschriebenen unteren und oberen Grenzwerten befindet, wobei die Blutdruckmesseinrichtung (8) eingerichtet ist, die Blutdruckmessung des Probanden auszulösen, wenn die Bestimmungseinrichtung (15) bestimmt, dass sich der gegenwärtig gemessene Wert der physiologischen Information sich zwischen den vorgeschriebenen unteren und oberen Grenzwerten befindet, wobei:

die Bestimmungseinrichtung (7, 15) eine Berechnungseinrichtung (71) aufweist, die eingerichtet ist, einen Durchschnittswert und eine Standardabweichung der gemessenen Werte der Reihe von Werten der physiologische Informationen, die vorher gespeichert sind, zu berechnen; und
wobei, wenn die gemessene physiologische Information zur Blutdruckmessung einen Wert liefert, der sich zwischen den vorgeschriebenen unteren und oberen Grenzwerten befindet, die von dem Durchschnittswert und der Standardabweichung, die von der Berechnungseinrichtung (71) berechnet werden, erhalten werden, die Bestimmungseinrichtung (7, 15) eingerichtet ist, zu bestimmen, dass sich der gelieferte Wert zwischen den vorgeschriebenen unteren und oberen Grenzwerten befindet und die Blutdruckmesseinrichtung (8) initiiert wird.

4. Blutdruckmessvorrichtung nach Anspruch 1, 2 oder 3, weiterhin umfassend:

eine Blutdruckspeichereinrichtung (11) zum Korrelieren und Speichern eines gemessenen Blutdruckwertes immer dann, wenn die Blutdruckmesseinrichtung (8) eine Messung liefert, einer zeitlichen Information, die angibt, wann der Blutdruck gemessen wird, und einer Information zu der spezifizierten Höhe, die durch die Bestimmungseinrichtung bestimmt wird; und
Anzeigeeinrichtung (3) zur Anzeige eines Inhalts der Blutdruckspeichereinrichtung (11).

5. Blutdruckmessverfahren, das die folgenden Schritte umfasst:

vorheriges Messen von physiologischer Information, die ein Blutsauerstoffsättigungsniveau ist (ST2, ST3);
diskontinuierliches oder kontinuierliches Speichern einer Reihe von Werten der physiologischen Information hinsichtlich deren Höhe wie zuvor gemessen für eine vorbestimmte Zeitperiode;
gegenwärtiges Messen von physiologischer Information, die ein Blutsauerstoffsättigungsniveau ist (ST102, ST103); und
Bestimmen, ob ein Wert der physiologischen Information, der gegenwärtig in dem Schritt des gegenwärtigen Messens gemessen wird, zum Bestimmen einer Auslösung der Messung des Blutdruckes des Probanden einen maximalen Schwellenwert überschreitet, kleiner als ein minimaler Schwellenwert ist, oder sich zwischen vorgeschriebenen oberen und unteren Grenzwerten befindet, wobei, wenn in dem Schritt des Bestimmens (ST104, ST105, ST106) bestimmt wird, dass der Wert der gegenwertig gemessenen physiologischen Information den maximalen Schwellenwert überschreitet, oder kleiner als der minimale Schwellenwert ist, oder sich zwischen den vorgeschriebenen oberen und unteren Grenzwerten befindet, eine Messung des Blutdrucks des Probanden beginnt, wobei:

der Schritt des Bestimmens ein Berechnen eines Durchschnittswertes und einer Standardabweichung der vorher gemessenen Werte der Reihe von Werten der physiologischer Informationen, die vorher gespeichert wurden, aufweist; und
wenn die gegenwärtig gemessene physiologische Information zur Bestimmung der Auslösung der Blutdruckmessung einen Wert liefert, der den maximalen Schwellenwert, der von dem Durchschnittswert und der Standardabweichung, die in dem Schritt des Berechnens berechnet werden, erhalten wird, überschreitet, wird in dem Schritt des Bestimmens bestimmt, dass der gelieferte Wert den maximalen Schwellenwert überschreitet, und eine Messung des Blutdrucks des Probanden beginnt, oder
wenn die gegenwärtig gemessene physiologische Information zur Bestimmung der Auslösung der Blutdruckmessung gegenwärtig einen Wert liefert, der kleiner ist als der minimale Schwellenwert, der von dem Durchschnittswert und der Standardabweichung, die in dem Schritt des Berechnens berechnet werden, erhalten wird, wird in dem Schritt des Bestimmens bestimmt, dass der gelieferte Wert kleiner als der minimale Schwellenwert ist und eine Messung des Blutdrucks des Probanden beginnt, oder
wenn die gegenwärtig gemessene physiologische Information zur Bestimmung der Auslösung der Blutdruckmessung gegenwärtig einen Wert liefert, der sich zwischen den vorgeschriebenen oberen und unteren Grenzwerten, die von dem Durchschnittswert und der Standardabweichung, die in dem Schritt des Berechnens berechnet werden, erhalten werden, befindet, wird in dem Schritt des Bestimmens bestimmt, dass sich der gelieferte Wert zwischen den vorgeschriebenen oberen und unteren Grenzwerten befindet und eine Messung des Blutdrucks des Probanden beginnt.

## Revendications

**1.** Appareil de mesure (1 ; 1A ; 1B ; 1C, 1D ; 1E ; 1F) de la pression artérielle comprenant :

des moyens de mesure (8) de la pression artérielle adaptés pour mesurer la pression artérielle d'un sujet ;
des moyens de mesure (6, 13, 14) d'information physiologique adaptés pour mesurer une information physiologique qui est le niveau de saturation en oxygène du sang ;
des moyens de stockage (12) d'information physiologique adapté pour stocker une série de valeurs de ladite information physiologique en niveau telle que mesurée par ledit moyen de mesure (6, 13, 14) d'information physiologique pendant une période de temps prédéterminée, lesdits moyens de stockage (12) d'information physiologique étant adaptés pour stocker de façon intermittente ou continue lesdites valeurs d'information physiologique en niveau telles que mesurées pendant ladite période de temps prédéterminée ; et
des moyens de détermination (7, 15) adaptés pour déterminer si une valeur de ladite information physiologique telle qu'actuellement mesurée par lesdits moyens de mesure (6, 13, 14) d'information physiologique pour détermination du lancement de la mesure de ladite pression artérielle du sujet dépasse une valeur seuil, dans lequel lesdits moyens de mesure (8) de pression artérielle sont adaptés pour, quand lesdits moyens de détermination (15) déterminent que la valeur de ladite information physiologique telle qu'actuellement mesurée dépasse la valeur seuil, être lancés afin de mesurer ladite pression artérielle du sujet, dans lequel :

lesdits moyens de détermination (7, 15) comprennent des moyens de calcul (71) adaptés pour calculer une valeur moyenne et un écart type de valeurs mesurées de ladite série d'information physiologique préalablement stockée ; et
quand ladite information physiologique actuellement mesurée pour détermination du lancement de ladite mesure de la pression artérielle fournit une valeur dépassant ladite valeur seuil obtenue à partir de ladite valeur moyenne et dudit écart type calculé par lesdits moyens de calcul (71), lesdits moyens de détermination (7, 15) sont adaptés pour déterminer que la valeur fournie dépasse la valeur seuil et lesdits moyens de mesure (8) de la pression artérielle sont lancés.

**2.** Appareil de mesure (1 ; 1A ; 1B ; 1C, 1D ; 1E ; 1F) de la pression artérielle comprenant :

des moyens de mesure (8) de la pression artérielle adaptés pour mesurer la pression artérielle d'un sujet ;
des moyens de mesure (6, 13, 14) d'infirmation physiologique adaptés pour mesurer une information physiologique qui est le niveau de saturation en oxygène du sang ;
des moyens de stockage (12) d'information physiologique adaptés pour stocker une série de valeurs de ladite information physiologique en niveau telle que mesurée par lesdits moyens de mesure (6, 13, 14) d'information physiologique pendant une période de temps prédéterminée, lesdits moyens de stockage (12) d'information physiologique étant adaptés pour stocker de façon intermittente ou continue lesdites valeurs d'information physiologique en niveau telles que mesurées pendant ladite période de temps prédéterminé et
des moyens de détermination (7, 15) adaptés pour déterminer si une valeur de ladite information physiologique telle qu'actuellement mesurée par ledit moyen de mesure (6, 13, 14) d'information physiologique pour détermination du lancement de la mesure de ladite pression artérielle du sujet est plus petite qu'une valeur seuil, dans lequel lesdits moyens de mesure (8) de pression artérielle sont adaptés pour, quand lesdits moyens de détermination (15) déterminent que la valeur de ladite information physiologique telle qu'actuellement mesurée est plus petite que la valeur seuil, être lancés afin de mesurer ladite pression artérielle du sujet, dans lequel :

lesdits moyens de détermination (7, 15) comprennent des moyens de calcul (71) adaptés pour calculer une valeur moyenne et un écart type de valeurs mesurées de ladite série d'information physiologique préalablement stockée ; et
quand ladite information physiologique actuellement mesurée pour détermination du lancement de ladite mesure de la pression artérielle fournit une valeur plus petite que ladite valeur seuil obtenue à partir de ladite valeur moyenne et dudit écart type calculé par lesdits moyens de calcul (71), lesdits moyens de détermination (7, 15) sont adaptés pour déterminer que la valeur fournie est plus petite que la valeur seuil et lesdits moyens de mesure (8) de la pression artérielle est lancé.

3. Appareil de mesure (1 ; 1A ; 1B ; 1C, 1D ; 1E ; 1F) de la pression artérielle comprenant :

des moyens de mesure (8) de la pression artérielle adaptés pour mesurer la pression artérielle d'un sujet ;
des moyens de mesure (6, 13, 14) d'information physiologique adaptés pour mesurer une information physiologique qui est le niveau de saturation en oxygène du sang ;
des moyens de stockage (12) d'information physiologique adaptés pour stocker une série de valeurs de ladite
information physiologique en niveau telle que mesurée par lesdits moyens de mesure (6, 13, 14) d'information
physiologique pendant une période de temps prédéterminée, lesdits moyens de stockage (12) d'information
physiologique étant adaptés pour stocker de façon intermittente ou continue lesdites valeurs d'information
physiologique en niveau telle que mesurées pendant ladite période de temps prédéterminé et
des moyens de détermination (7, 15) adaptés pour déterminer si une valeur de ladite information physiologique
telle qu'actuellement mesurée par lesdits moyens de mesure (6, 13, 14) d'information physiologique pour détermination du lancement de la mesure de ladite pression artérielle du sujet est comprise entre des limites
supérieure et inférieure déterminées, dans lequel lesdits moyens de mesure (8) de pression artérielle sont
adaptés pour, quand lesdits moyens de détermination (15) déterminent que la valeur de ladite information
physiologique telle qu'actuellement mesurée est comprise entre les limites supérieure et inférieure déterminées,
être lancé afin de mesurer ladite pression artérielle du sujet, dans lequel :

lesdits moyens de détermination (7, 15) comprennent des moyens de calcul (71) adaptés pour calculer
une valeur moyenne et un écart type de valeurs mesurées de ladite série d'information physiologique
préalablement stockée ; et
quand ladite information physiologique mesurée pour ladite mesure de la pression artérielle fournit une
valeur comprise entre les limites supérieure et inférieure déterminées obtenue à partir de ladite valeur
moyenne et dudit écart type calculé par lesdites moyens de calcul (71), lesdits moyens de détermination
(7, 15) sont adaptés pour déterminer que la valeur fournie est comprise entre les limites supérieure et
inférieure déterminées et lesdits moyens de mesure (8) de la pression artérielle sont lancés.

4. Appareil de mesure de la pression artérielle selon la revendication 1, 2 ou 3, comprenant en outre :

des moyens de stockage (11) de pression artérielle corrélant et stockant une valeur de pression artérielle à
chaque fois que lesdits moyens de mesure (8) de la pression artérielle fournit une mesure, une information
temporelle indiquant le moment où la pression artérielle est mesurée et une information dudit niveau spécifié
déterminé par lesdits moyens de détermination : et
des moyens d'affichage (3) affichant un contenu desdits moyens de stockage (11) de pression artérielle.

5. Procédé de mesure de la pression artérielle comprenant les étapes consistant à :

mesurer préalablement une information physiologique qui est le niveau de saturation en oxygène du sang (ST2,
ST3) ;
stocker de façon intermittente ou continue une série de ladite information physiologique en niveau telle que
préalablement mesurée pendant une période de temps prédéterminée ;
mesurer actuellement une information physiologique qui est le niveau de saturation en oxygène du sang (ST2,
ST3) ; et
déterminer si une valeur de ladite information physiologique telle qu'actuellement mesurée à l'étape de mesure
actuelle pour détermination du lancement de la mesure de ladite pression artérielle du sujet dépasse une valeur
seuil maximale, est plus petite qu'une valeur seuil minimale, ou bien est comprise entre des limites supérieure
et inférieure déterminées, dans lequel, quand une décision est prise à l'étape de détermination (ST104, ST105,
ST106) que la valeur de ladite information physiologique telle qu'actuellement mesurée dépasse la valeur seuil
maximale, ou est plus petite que la valeur seuil minimale, ou bien est comprise entre lesdites limites supérieure
et inférieure déterminées, une mesure de ladite pression artérielle du sujet démarre, dans lequel :

ladite étape de détermination comprend le calcul d'une valeur moyenne et d'un écart type de valeurs
préalablement mesurées de ladite série d'information physiologique préalablement stockée ; et
quand ladite information physiologique actuellement mesurée pour détermination du lancement de ladite
mesure de la pression artérielle fournit une valeur dépassant ladite valeur seuil maximale obtenue à partir
de ladite valeur moyenne et dudit écart type calculés à ladite étape de calcul, une décision est prise à l'étape de détermination que la valeur fournie dépasse ladite valeur seuil maximale et une mesure de ladite pression artérielle du sujet démarre, ou

quand ladite information physiologique actuellement mesurée pour détermination du lancement de ladite mesure de la pression artérielle fournit une valeur plus petite que ladite valeur seuil minimale obtenue à partir de ladite valeur moyenne et dudit écart type calculés à ladite étape de calcul, une décision est prise à l'étape de détermination que la valeur fournie est plus petite que ladite valeur seuil minimale et une mesure de ladite pression artérielle du sujet démarre, ou

quand ladite information physiologique actuellement mesurée pour détermination du lancement de ladite mesure de la pression artérielle fournit une valeur comprise entre lesdites limites supérieure et inférieure déterminées obtenue à partir de ladite valeur moyenne et dudit écart type calculés à ladite étape de calcul, une décision est prise à l'étape de détermination que la valeur fournie est comprise entre lesdites limites supérieure et inférieure prescrites et une mesure de ladite pression artérielle du sujet démarre.

## FIG.1A

## FIG.1B

FIG.2

FIG.3

## FIG.4

```
          ┌──────────────────────────┐
          │  PULSE RATE STORAGE      │  (DURING SLEEP)
          │  OPERATION (START)       │
          └──────────────────────────┘
                      │
                      ▼
ST1 ─────       START
              OPERATION?            ── NO
                      │ YES
                      ▼
ST2 ─────  ┌──────────────────────────┐
           │   DETECT PULSE WAVE      │
           └──────────────────────────┘
                      │
ST3 ─────  ┌──────────────────────────┐
           │   CALCULATE PULSE RATE   │
           └──────────────────────────┘
                      │
ST4 ─────  ┌──────────────────────────┐
           │   STORE PULSE RATE       │
           └──────────────────────────┘
                      │
                      ▼
ST5 ─────       STOP
              OPERATION?             ── NO
                      │ YES
                      ▼
ST6 ─────  ┌──────────────────────────────────────────┐
           │  CALCULATE MAXIMUM, MINIMUM, AVERAGE      │
           └──────────────────────────────────────────┘
                      │
                      ▼
                  ┌─────────┐
                  │   END   │
                  └─────────┘
```

## FIG.5

```
        ┌─────────────────────────┐
        │ BLOOD PRESSURE          │
        │ MEASUREMENT OPERATION   │
        │ (START)                 │
        └─────────────────────────┘
                    │
ST101 ─┐       ◇ START
        │        OPERATION? ────── NO
                    │ YES
ST102 ─┐       ┌─────────────────────┐
        │      │ DETECT PULSE WAVE   │
        └      └─────────────────────┘
ST103 ─┐       ┌─────────────────────┐
        │      │ CALCULATE PULSE RATE│
        └      └─────────────────────┘
ST104 ─┐       ◇ CLOSE TO MAX.? ──── YES
                    │ NO
ST105 ─┐       ◇ CLOSE TO MIN.? ──── YES
                    │ NO
ST106 ─┐   NO ── ◇ CLOSE TO AVERAGE?
                    │ YES
ST107 ─┐       ┌─────────────────────┐
        │      │ MEASURE BLOOD PRESSURE│
        └      └─────────────────────┘
ST108 ─┐       ┌─────────────────────┐
        │      │ STORE BLOOD PRESSURE│
        └      │ VALUE AND DATE & TIME│
               └─────────────────────┘
ST109 ─┐       ◇ STOP
        │         OPERATION? ────── NO
                    │ YES
               ┌─────────┐
               │  END    │
               └─────────┘
```

FIG.6

| PULSE RATE | BLOOD PRESSURE | TIME |
|---|---|---|
| MAXIMUM VALUE (>102) | 162 / 95 | 7:06 AM |
| | 158 / 94 | 7:23 AM |
| AVERAGE VALUE (76~86) | 142 / 74 | 0:15 AM |
| | 140 / 76 | 5:20 AM |
| | 144 / 78 | 5:25AM |
| MINIMUM VALUE (<58) | 123 / 68 | 3:12 AM |
| | 118 / 65 | 3:20 AM |

PULSE RATE AVERAGE : 80    STANDARD DEVIATION : 11

FREQUENCY

58    80    102    PULSE RATE

FIG.7

CONSOLE — 4

DISPLAY UNIT — 3

1A

2

CPU — 7

CALCULATOR — 71

UPPER ARM CUFF — 5

BLOOD PRESSURE MONITOR — 8

16

BLOOD PRESSURE STORAGE — 11

TIMER — 9

FINGER CUFF — 6

PULSE WAVE DETECTOR

OXYGEN SATURATION LEVEL CALCULATOR — 14A

COMPARATOR & DETERMINER — 10

15A

13

OXYGEN SATURATION LEVEL STORAGE — 12A

# FIG.8

# FIG.9

## FIG.10

FIG.11

FIG.12

**EP 1 568 314 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8131408 A
- JP 62155829 A
- JP 2023940 A
- EP 1127538 A

**Non-patent literature cited in the description**

- **CALVO C et al.** Further evaluation of the ambulatory monitoring pressor effect (''ABPM effect'') in patients with essential hypertension. *J. Am. Hypertension,* 2003, vol. 16 (5
- **LOPEZ JE.** Implications of the ''ABPM effect'' in hypertensive patients: Changes in the dipping pattern of blood pressure variability. *J. Am. Hypertension,* 2003, vol. 16 (5
- **HERMIDA RC.** Sampling requirements for ambulatory blood pressure monitoring in the diagnosis of hypertension. *J. Am. Hypertension,* 2003, vol. 16 (5
- **STENEHJEM AE.** Gender and age differences in variability of ambulatory daytime pulse pressure. *J. Am. Hypertension,* 2003, vol. 16 (5
- **MURAKAMI.** Novelty Effect, Holiday Effect and Weekly Variation of 7-day Ambulatory (24-Hour) BP of Hypertensives. *Circulation Journal, Japanese Circulation Society,* 2003, vol. 67